# EUROPEAN PATENT APPLICATION

(11) **EP 1 829 576 A1**
(43) Date of publication of application: **05.09.2007**
(21) Application number: 05820184.9
(22) Date of filing: 21.12.2005
(51) Int. Cl.: A61M 5/145

(54) **CHEMICAL LIQUID INJECTION DEVICE**

(30) Priority: 24.12.2004 JP 2004373278
(71) Applicant: Nemoto Kyorindo Co., Ltd., Tokyo 113-0033 (JP)
(72) Inventor: HACHIYA, Takashi c/o Nemoto Kyorindo Co., Ltd., Tokyo 1130033 (JP); NEMOTO, Shigeru c/o Nemoto Kyorindo Co., Ltd., Tokyo 1130033 (JP)
(74) Representative: Gill, David Alan
(86) International application number: PCT/JP2005/023471
(87) International publication number: WO 2006/068171

(57) **Abstract**

In a chemical solution injector, an injection head to which a chemical solution syringe is attached is rotatable between a preparation attitude where the leading end of the injection head is oriented upward, and, an injection attitude where the leading end is oriented downward, and these attitude are determined by an attitude -determining means (122) to operate and control a syringe-driving mechanism (117) that drives a chemical solution syringe (200). First, initial state is made in the device such that only chemical solution suction is freely operable. Then, the device is set, when the chemical solution suction is completed and the injection head (110) is in preparation attitude, to a state where only bubble removal is performable, and is set, when the bubble removal is completed and the injection head (110) is the injection attitude , to a state where only chemical solution injection is performable. Chemical solution injection with a chemical solution syringe in which bubble removal has not been complete can be automatically prevented.

## Description

### Technical Field

The present invention relates to a chemical solution injector for injecting a chemical solution into a patient by relatively moving a cylinder member and a piston member of a chemical solution syringe, and more particularly, to a chemical solution injector including a vertically pivotable injection head on which a chemical solution syringe is mounted.

### Background Art

Presently available imaging diagnostic apparatuses for capturing diagnostic images of patients include CT (Computed Tomography) scanners, MRI (Magnetic Resonance Imaging) apparatuses, PET (Positron Emission Tomography) apparatuses, ultrasonic diagnostic apparatuses, CT angiography apparatuses, MRA (MR angiography) apparatuses and the like.

When the abovementioned imaging diagnostic apparatuses are used, a chemical solution such as a contrast medium and physiological saline may be injected into a patient. Chemical solution injectors for automatically performing the injection have been put into practical use. Such a chemical solution injector has a syringe-driving mechanism formed of a driving motor, a slider mechanism and the like, for example. A chemical solution syringe is removably mounted on an injection head that contains the syringe-driving mechanism.

The chemical solution syringe includes a cylinder member and a piston member slidably inserted into the cylinder member. There are a pre-filled type and a refill type in the chemical solution syringe. The chemical solution syringe of the pre-filled type includes a cylinder member filled with a chemical solution and is wholly sealed by a packing material for shipment. The chemical solution syringe of the refill type includes a cylinder member which can be filled with a desired chemical solution by a user.

In order to inject a chemical solution into a patient from a chemical solution syringe of the pre-filled type, the chemical solution syringe connected to an injection tube is mounted on an injection head, which is positioned in an injection attitude in which its leading end is located below its trailing end, for example. Since the leading end of the chemical solution syringe connected to the injection tube is also located below in the injection attitude, bubble mixed into the chemical solution at the time of connection of the injection tube stays at the trailing end of the chemical solution syringe.

Then, the injection head is manually turned, together with the chemical solution syringe mounted thereon, to a preparation attitude in which the leading ends of the chemical solution syringe and the injection head are located above. In the preparation attitude, the bubbles mixed in the chemical solution stays at the leading end of the chemical solution syringe. If the syringe-driving mechanism is activated in this state to press the piston member into the cylinder member to a predetermined position, the bubbles are discharged from the chemical solution syringe and from the injection tube together with a slight amount of the chemical solution.

The injection tube is connected to a blood vessel of a patient in this state, and the injection head is manually turned together with the chemical solution syringe mounted thereon to the injection attitude. The syringe-driving mechanism is activated in this state to press the piston member into the cylinder member to inject the chemical solution in the chemical solution syringe into the patient through the injection tube. Even if small bubbles are present in the chemical solution in the chemical solution syringe, the bubbles stay at the trailing end of the chemical solution syringe and thus bubbles will not mixed into the chemical solution which is injected into the patient from the leading end of the chemical solution syringe.

To inject a chemical solution into a patient from a chemical solution syringe of the refill type, a large chemical solution tank filled with the chemical solution is connected through a dedicated suction tube to the chemical solution syringe, which is mounted on an injection head positioned in an injection attitude. The injection head is manually turned to a preparation attitude, and a syringe-driving mechanism is activated to pull a piston member out of a cylinder member to a predetermined position.

This causes the chemical solution in the chemical solution tank to be sucked into the chemical solution syringe through the suction tube. After the suction is completed, the suction tube and the chemical solution tank are disconnected from the chemical solution syringe. Then, an injection tube is connected to the chemical solution syringe. The syringe-driving mechanism is activated to a predetermined position to discharge bubbles mixed in the chemical solution in the chemical solution syringe. Thereafter, injection operation is performed in the same manner as in the chemical solution syringe of the pre-filled type.

In some chemical solution injectors including the vertically turnable or pivotable injection head as described above, the inclination of the injection head is detected to control the chemical solution injection such that the injection head can be activated in accordance with manual operation only when the leading end of the chemical solution syringe is located below (see, for example, patent document 1 below).

In such a chemical solution injector, however, the injection head is deactivated in the preparation attitude, which makes it impossible to favorably remove bubbles and to suck the chemical solution into the chemical solution syringe of the refill type.

To address this, a proposal has been made in which the piston member is moved forward and rearward when the leading end of the chemical solution syringe is located above and the piston member is moved only forward, when the leading end is located below, and after bubble is discharged from the chemical solution syringe to the solution tank, the chemical solution is sucked into the chemical solution syringe and is injected from the chemical solution syringe into the patient (see, for example, patent document 2 below).
Patent Document 1: Japanese Patent Laid-Open No. 10(1998)-165396
Patent Document 2: Japanese Patent Application No. 2002-508503 (WO2002/004049

In the chemical solution injectors of Patent Documents 1 and 2 described above, since the operation of the injection head is only controlled in accordance with the attitude, actually, it is difficult to prevent inappropriate operation reliably.

For example, in the injection attitude of the injection head, not only the injection from the syringe into the patient but also the mounting and demounting of the syringe are typically performed. If the injection head can be activated in the injection attitude, the chemical solution may be injected into the patient from the chemical solution syringe containing bubbles which are not completely removed. Especially in the chemical solution syringe of the refill type, air may be injected into the patient from the chemical solution syringe which has not sucked the chemical solution yet.

To solve the abovementioned problems, in a chemical solution injector proposed by the present applicant, only bubble-removing and chemical solution suction are possible when the mounting of a chemical solution syringe is detected. Chemical solution injection is possible after an operator visually recognizes the removal of bubbles and then manually operates a dedicated release switch. This prevents injection of the chemical solution or air into a patient from the chemical solution syringe containing bubbles which are not completely removed. However, this requires the dedicated release switch or the like additionally provided for the chemical solution injector, and also causes an increased burden in operation such as the visual recognition and switch operation.

### Disclosure of the Invention

The present invention has been made in view of the abovementioned problems, and it is an object thereof to provide a chemical solution injector, which prevents chemical solution injection from being performed with a chemical solution syringe containing bubbles which are not completely removed, without requiring an additionally provided release switch or an increased burden in operation.

The chemical solution injector according to a first aspect of the present invention injects a chemical solution into a patient from a chemical solution syringe by relatively moving a cylinder member and a piston member of the chemical solution syringe, the cylinder member having a conduit portion formed at its leading end and an opening formed at its trailing end, the piston member being slidably inserted into the cylinder member through the opening. The chemical solution injector includes an injection head, a syringe-driving mechanism, a head support mechanism, an attitude-determining means , an bubble-removing means, a chemical solution injecting means, an initial setting means, a removal-control means, and an injection-control means.

The chemical solution syringe is removably mounted on the injection head. The syringe-driving mechanism relatively moves the cylinder member and the piston member of the chemical solution syringe mounted on the injection head. The head support mechanism supports the injection head movable to an injection attitude in which a leading end of the chemical solution syringe is located below a trailing end thereof, and to a preparation attitude in which the leading end is located above the trailing end. The attitude determining means determines whether the injection head is positioned in the injection attitude or the preparation attitude. The bubble-removing means presses the piston member into the cylinder member for a predetermined distance under control of the syringe-driving mechanism to remove bubbles from the chemical solution in the chemical solution syringe. The chemical solution injecting means presses the piston member into the cylinder member under control of the syringe-driving mechanism to inject the chemical solution into the patient from the chemical solution syringe. The initial setting means performs initial setting such that the bubble-removing means and the chemical solution injecting means are inoperable. The removal-control means causes the bubble-removing means to be operable while the chemical solution injecting means is held inoperable when it is determined that the injection head is positioned in the preparation attitude after the completion of the initial setting. The injection-control means causes the bubble-removing means to be inoperable and causes the chemical solution injecting means to be operable when it is determined that the injection head is positioned in the injection attitude after the completion of the operation of the bubble-removing means in the preparation attitude.

Thus, in the chemical solution injector according to the first aspect of the present invention, in the initial state, the bubble-removing or the chemical solution injection cannot be performed. When the injection head is positioned in the preparation attitude after the initial state, only the bubble-removing can be performed for the chemical solution syringe. When the injection head is positioned in the injection attitude after the completion of the bubble-removing, only the chemical solution injection can be performed.

The chemical solution injector according to a second aspect of the present invention includes an injection head, a syringe-driving mechanism, a head support mechanism, an attitude-determining means , a chemical solution sucking means, an bubble-removing means, a chemical solution injecting means, an initial setting means, a removal-control means, and an injection-control means. The chemical solution sucking means pulls the piston member from the cylinder member for a predetermined distance under control of the syringe-driving mechanism to suck the chemical solution into the chemical solution syringe from the solution tank. The initial setting means performs initial setting such that the bubble-removing means and the chemical solution injecting means are inoperable. The removal-control means causes the chemical solution sucking means and the chemical solution injecting means to be inoperable and causes the bubble-removing means to be operable, when it is determined that the injection head is positioned in the preparation attitude after the completion of the operation of the chemical solution sucking means. The injection-control means causes the chemical solution sucking means and the bubble-removing means to be inoperable and causes the chemical solution injecting means to be operable when it is determined that the injection head is positioned in the injection attitude after the operation of the chemical solution sucking means and the operation of the bubble-removing means are completed in order.

Thus, in the chemical solution injector according to the second aspect of the present invention, in the initial state, only the chemical solution suction can be performed. When the injection head is positioned in the preparation attitude after the completion of the chemical solution suction, only the bubble-removing can be performed. When the injection head is positioned in the injection attitude after the completion of the bubble-removing, only the chemical solution injection can be performed.

The chemical solution injector according to a third aspect of the present invention includes an injection head, a syringe-driving mechanism, a syringe-determining means, a head support mechanism, an attitude-determining means , a chemical solution sucking means, an bubble-removing means, a chemical solution injecting means, an initial setting means, a pre-filled removing means, a pre-filled injecting means, a refill sucking means, a refill removing means, and a refill injecting means.

The syringe-determining means determines whether the chemical solution syringe mounted on the injection head is of the pre-filled type or the refill type. The pre-filled removing means causes the bubble-removing means to be operable, and causes the chemical solution injecting means and the chemical solution sucking means inoperable, when it is determined that the injection head is positioned in the preparation attitude and the chemical solution syringe is of the pre-filled type. The pre-filled injecting means causes the chemical solution sucking means and the bubble-removing means to be inoperable, and causes the chemical solution injecting means to be operable, when it is determined that the injection head is positioned in the injection attitude and that the chemical solution syringe is of the pre-filled type after the completion of the operation of the bubble-removing means in the preparation means. The refill sucking means initially causes the chemical solution injecting means and the bubble-removing means to be inoperable and causes the chemical solution sucking means to be operable when it is determined that the chemical solution syringe is of the refill type after the completion of the initial setting. The refill removing means causes the bubble-removing means to be operable, and causes the chemical solution injecting means to be inoperable, when it is determined that the injection head is positioned in the preparation attitude and that the chemical solution syringe is of the refill type after the completion of the operation of the chemical solution sucking means. The refill injecting means causes the chemical solution sucking means and the bubble-removing means to be inoperable and causes the chemical solution injecting means to be operable, when it is determined that the injection head is positioned in the injection attitude and that the chemical solution syringe is of the refill type after the operation of the chemical solution sucking means and the operation of the bubble-removing means are completed.

Thus, in the chemical solution injector according to the third aspect of the present invention, in the initial state, the chemical solution injection cannot be performed. For the chemical solution syringe of the pre-filled type, when the injection head is positioned in the preparation attitude after the initial state, only the bubble-removing can be performed, and when the injection head is positioned in the injection attitude after the completion of the bubble-removing, only the chemical solution injection can be performed. For the chemical solution syringe of the refill type, when the injection head is positioned in the preparation attitude after the completion of the chemical solution suction after the initial state, only the bubble-removing can be performed, and when the injection head is positioned in the injection attitude after the completion of the bubble-removing, only the chemical solution injection can be performed.

The chemical solution injector according to a fourth aspect of the present invention includes an injection head, a syringe-driving mechanism, a head support mechanism, an attitude-determining means , a chemical solution injecting means, an initial setting means, a manual-operating means, an operation-driving means, a completion detecting means, and an injection-control means. The initial setting means performs initial setting such that the chemical solution injecting means is inoperable. The manual-operating means receives entry-operation to cause the syringe-driving mechanism to perform at least bubble-removing of removing bubbles from the chemical solution in the chemical solution syringe in real time. The operation-driving means operates the syringe-driving mechanism in real time in response to the entry-operation to the manual-operating means when it is determined that the injection head is positioned in the preparation attitude. The completion detecting means detects the completion of the bubble-removing based on at least one of a moving distance and a position of the syringe-driving mechanism when it is determined that the injection head is positioned in the preparation attitude. The injection-control means causes the chemical solution injecting means to be operable when it is determined that the injection head is positioned in the injection attitude after the completion of the bubble-removing is detected.

Thus, in the chemical solution injector according to the fourth aspect of the present invention, the chemical solution injection cannot be performed in the initial state. When the injection head is positioned in the preparation attitude after the initial state, the bubble-removing is performed for the chemical solution syringe in real time in response to the entry-operation. When the injection head is positioned in the injection attitude after the completion of the bubble-removing, only the chemical solution injection can be performed.

The chemical solution injector according to a fifth aspect of the present invention includes an injection head, a syringe-driving mechanism, a head support mechanism, an attitude-determining means, a chemical solution injecting means, an initial setting means, a manual-operating means, an operation-driving means, a suction-detecting means, a removal detecting means, and an injection-control means. The manual-operating means receives entry-operation to cause the syringe-driving mechanism to perform at least chemical solution suction of sucking the chemical solution into the chemical solution syringe from the solution tank and bubble-removing of removing bubbles from the chemical solution in the chemical solution syringe in real time. The suction-detecting means detects the completion of the chemical solution suction based on at least one of a moving distance and a position of the syringe-driving mechanism after the completion of the initial setting. The removal detecting means detects the completion of the bubble-removing based on at least one of a moving distance and a position of the syringe-driving mechanism when it is determined that the injection head is positioned in the preparation attitude after the completion of the chemical solution suction. The injection-control means causes the chemical solution injecting means to be operable when it is determined that the injection head is positioned in the injection attitude after the completion of the chemical solution suction and the completion of the bubble-removing are detected in order.

Thus, in the chemical solution injector according to the fifth aspect of the present invention, the chemical solution suction is performed in real time in response to the entry-operation in the initial state. When the injection head is positioned in the preparation attitude after the completion of the chemical solution suction, only the bubble-removing can be performed in real time in response to the entry-operation. When the injection head is positioned in the injection attitude after the completion of the bubble-removing, only the chemical solution injection can be performed.

The chemical solution injector according to a sixth aspect of the present invention includes an injection head, a syringe-driving mechanism, a head support mechanism, an attitude-determining means , a chemical solution injecting means, an initial setting means, a manual-operating means, an operation-driving means, a suction-detecting means, a removal detecting means, a syringe-determining means, and an injection-control means. The syringe-determining means determines whether the chemical solution syringe mounted on the injection head is of the pre-filled type or the refill type. The injection-control means causes the chemical solution injecting means to be operable after the completion of the bubble-removing is detected when it is determined that the chemical solution syringe is of the pre-filled type and causes the chemical solution injecting means to be operable after the completion of the chemical solution suction and the completion of the bubble-removing are detected in order when it is determined that the chemical solution syringe is of the refill type.

Thus, in the chemical solution injector according to the sixth aspect of the present invention, the chemical solution injection cannot be performed in the initial state. For the chemical solution syringe of the pre-filled type, when the injection head is positioned in the preparation attitude after the initial state, the bubble-removing is performed in real time in response to the entry-operation, and when the injection head is positioned in the injection attitude after the completion of the bubble-removing, only the chemical solution injection can be performed. For the chemical solution syringe of the refill type, the chemical solution suction is performed in real time in response to the entry-operation after the initial state, and when the injection head is positioned in the preparation attitude after the completion of the chemical solution suction, only the bubble-removing can be performed in real time in response to the entry-operation, and when the injection head is positioned in the injection attitude after the completion of the bubble-removing, only the chemical solution injection can be performed.

Various means referred to in the present invention may be arranged to perform their functions, and may comprise dedicated hardware for performing a predetermined function, a data processing apparatus whose predetermined function is given by a computer program, a predetermined function performed in a data processing apparatus according to a computer program, or a combination thereof.

Various components referred to in the present invention do not need to be a separate entity. A plurality of components may be constructed as one member, a certain component may be part of another component, or a certain component may have a portion overlapping a portion of another component.

### Effect of the Invention

According to the present invention, the followings can be prevented automatically without requiring an additionally provided release switch or an increased burden in operation:
To perform the chemical solution injection in the chemical solution syringe for which the bubble-removing is not been completed in the chemical solution injector according to the first aspect of the present invention;
To perform the bubble-removing in the chemical solution syringe for which the chemical solution suction is not completed, and to perform the chemical solution injection in the chemical solution syringe for which the bubble-removing is not completed in the chemical solution injector according to the second aspect of the present invention;
To perform the chemical solution injection in the chemical solution syringe of the pre-filled type for which the bubble-removing is not completed, to perform the bubble-removing in the chemical solution syringe of the refill type for which the chemical solution suction is not completed, and to perform the chemical solution injection in the chemical solution syringe of the refill type for which the bubble-removing is not completed in the chemical solution injector according to the third aspect of the present invention;
To perform the chemical solution injection in the chemical solution syringe for which the bubble-removing is not complete in the chemical solution injector according to the fourth aspect of the present invention;
To perform the bubble-removing in the chemical solution syringe for which the chemical solution suction is not completed and to perform the chemical solution injection in the chemical solution syringe for which the bubble-removing is not completed in the chemical solution injector according to the fifth aspect of the present invention; and
To perform the chemical solution injection in the chemical solution syringe of the pre-filled type for which the bubble-removing is not completed, to perform the bubble-removing in the chemical solution syringe of the refill type for which the chemical solution suction is not completed, and to perform the chemical solution injection in the chemical solution syringe of the refill type for which the bubble-removing is not completed in the chemical solution injector according to the sixth aspect of the present invention.

### Brief Description of the Drawings

Fig. 1 is a schematic block diagram showing the logical structure of a chemical solution injector according to an embodiment of the present invention;
Fig. 2 is a block diagram showing the circuit structure of an imaging diagnostic system ;
Fig. 3 is a perspective view showing the outer appearance of the chemical solution injector;
Fig. 4 is a perspective view showing how to mount a chemical solution syringe on an injection head of the chemical solution injector with a pressure-resistant cover;
Fig. 5 is a perspective view showing the outer appearance of the imaging diagnostic system;
Fig. 6(a) is a side view showing the injection head in an injection attitude and Fig. 6(b) is a side view showing the injection head in a preparation attitude; and
Fig. 7 is a flow chart showing the processing operation in the chemical solution injector.

### Description of Reference Numerals

- 100: CHEMICAL SOLUTION INJECTOR
- 110: INJECTION HEAD
- 112: MOVABLE ARM SERVING AS HEAD SUPPORT MECHANISM
- 117: SYRINGE-DRIVING MECHANISM
- 120: MOUNT/DEMOUNT DETECTION SENSOR SERVING AS MOUNT/DEMOUNT DETECTING MEANS
- 122: INCLINATION DETECTION SENSOR SERVING AS ATTITUDE-DETERMINING MEANS
- 141: CHEMICAL SOLUTION SUCKING MEANS
- 142: BUBBLE-REMOVING MEANS
- 143: CHEMICAL SOLUTION INJECTING MEANS
- 144: INITIAL SETTING MEANS
- 145: REMOVAL-CONTROL MEANS
- 146: INJECTION-CONTROL MEANS
- 147: DRIVE-REGULATING MEANS
- 200: CHEMICAL SOLUTION SYRINGE
- 210: CYLINDER MEMBER
- 212: CONDUIT
- 220: PISTON MEMBER
- 1000: IMAGING DIAGNOSTIC SYSTEM

### Best Mode for Carrying Out the Invention

### [Configuration of Embodiment]

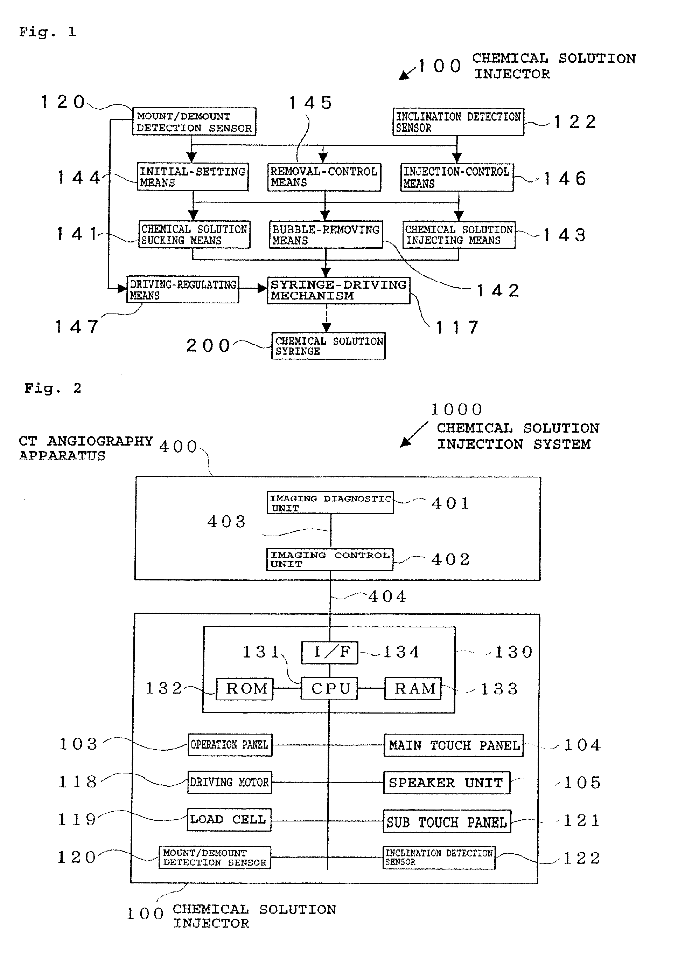

An embodiment of the present invention will hereinafter be described with reference to Figs. 1 to 7. As shown in Figs. 1 to 3, imaging diagnostic system 1000 of the embodiment according to the present invention includes chemical solution injector 100, chemical solution syringe 200, CT angiography apparatus 400 serving as an imaging diagnostic apparatus, and pressure-resistant cover 300 serving as a cylinder adapter, and injects a contrast medium or the like as a chemical solution into a patient (not shown), as described later in detail.

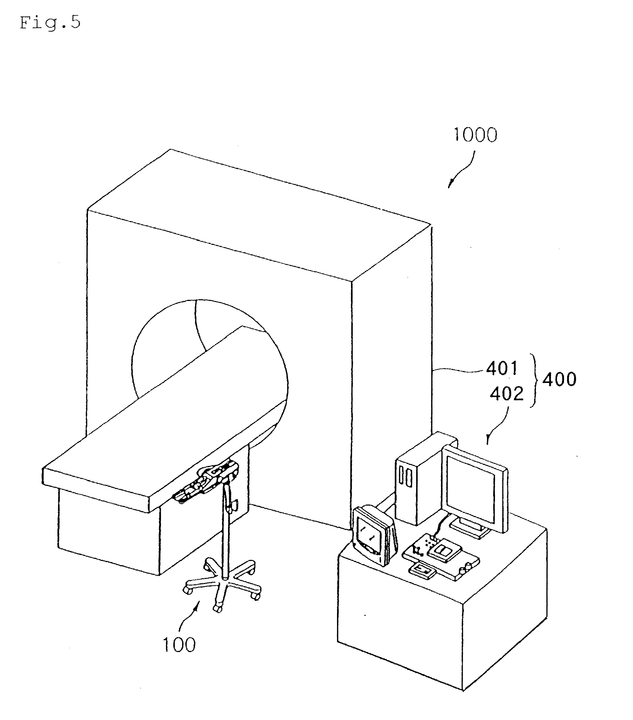

As shown in Fig. 5, CT angiography apparatus 400 includes imaging diagnostic unit 401 serving as a mechanism for performing imaging and imaging control unit 402 such that imaging diagnostic unit 401 and imaging control unit 402 are wire-connected through communication network 403. Imaging diagnostic unit 401 produces diagnostic images of a patient. Imaging control unit 402 controls the operation of imaging diagnostic unit 401.

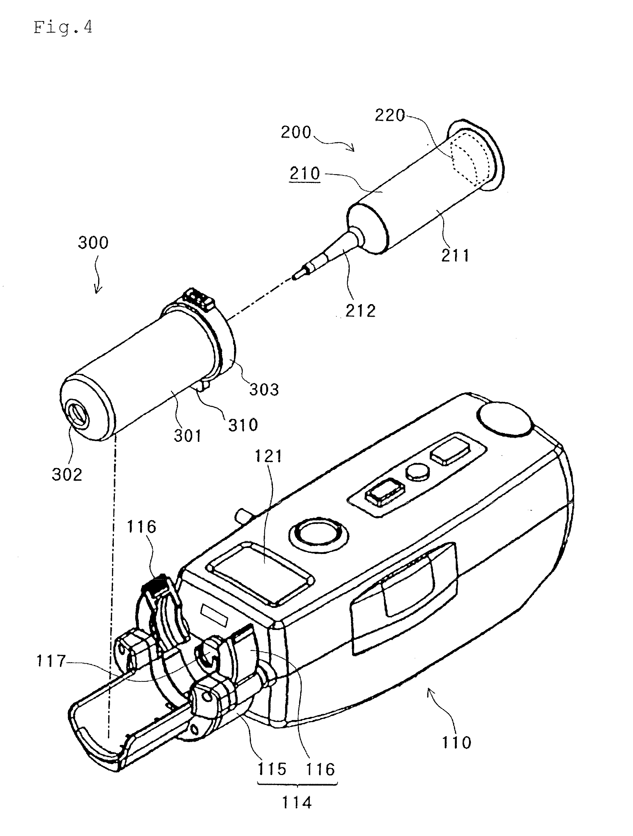

As shown in Fig. 4, chemical solution syringe 200 comprises cylinder member 210 and piston member 220 wherein piston member 220 is slidably inserted into cylinder member 210. Cylinder member 210 includes cylindrical hollow body 211 which has conduit 212 formed at its closed leading end surface. The trailing end of body 211 of cylinder member 210 is opened, and piston member 220 is inserted from the opening into the interior of body 211.

Pressure-resistant cover 300 also includes cylindrical hollow body 301 which has through-hole 302 at its closed leading end surface. The trailing end of pressure-resistant cover 300 is opened, and cylinder member 210 of chemical solution syringe 200 is inserted from the opening into the interior of body 301. Pressure-resistant cover 300 has annular cover flange 303 integrally formed on the outer circumference of the trailing end. Magnetic material 310 is contained in a convex portion of a lower section of cover flange 303.

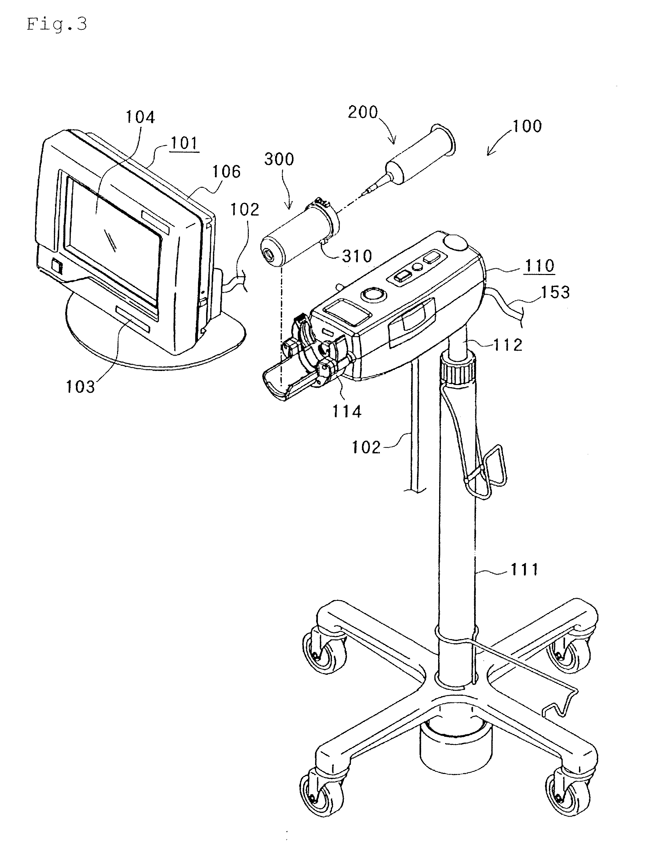

As shown in Fig. 3, chemical solution injector 100 of the embodiment includes injection control unit 101 and injection head 110 which are constructed as separate components. Injection control unit 101 and injection head 110 are wire-connected through communication cable 102. Injection head 110 is attached to the top end of caster stand 111 by movable arm 112 serving as a head support mechanism. Head body 113 of injection head 110 has flange holding mechanism 114 on the front surface thereof for removably holding pressure-resistant cover 300 together with chemical solution syringe 200.

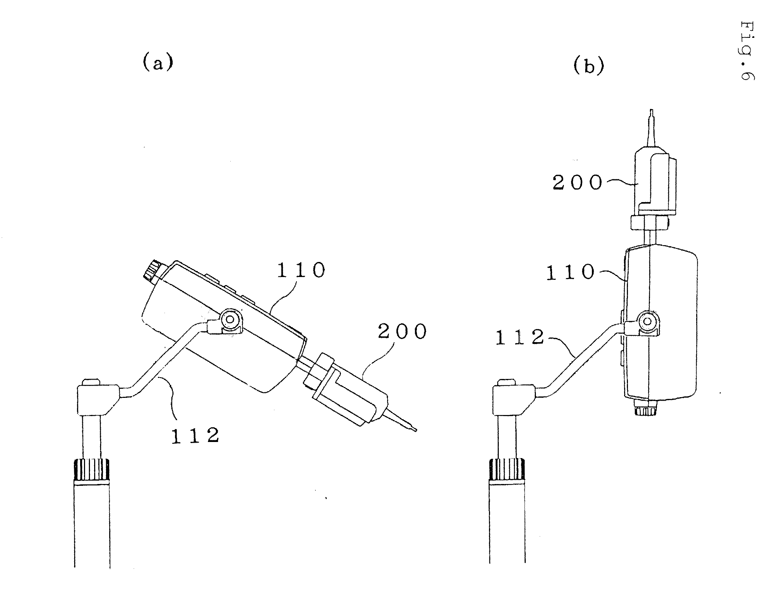

Movable arm 112 supports injection head 110 to be pivotable vertically. Injection head 110 is supported in an injection attitude in which the leading end of chemical solution syringe 200 mounted thereon is located below the trailing end as shown in Fig. 6(a). And, injection head 110 is supported in a preparation attitude in which the leading end is located above the trailing end as shown in Fig. 6(b).

As shown in Fig. 4, flange holding mechanism 114 is formed of semicircular fixed holding member 115 fixed to a lower portion of the front surface of head body 113 and a pair of movable holding members 116 individually supported to be pivotable upward and downward at the left and right ends of fixed holding member 115. Cover flange 303 of pressure-resistance cover 300 is fitted into fixed holding member 115 from above, and the upper portion of cover flange 303 is held from above by paired movable holding members 116 on the left and right.

Flange holding mechanism 114 has a concave portion (not shown) formed in a lower section on the inner side, and magnetic material 310 of cover flange 303 is removably inserted thereinto. The concave portion of flange holding mechanism 114 contains mount/demount detection sensor 120 formed of a hall device or the like, serving as a mount/demount detecting means, to detect magnetic material 310. When chemical solution syringe 200 is mounted on injection head 110 together with pressure-resistant cover 300, the mounting of chemical solution syringe 200 is detected by mount/demount detection sensor 120.

Injection head 110 has syringe-driving mechanism 117 placed on the front surface at the center on the inner side of flange holding mechanism 114. Syringe-driving mechanism 117 holds and presses piston member 220 of chemical solution syringe 200 into cylinder member 210. Syringe-driving mechanism 117 includes driving motor 118 as a driving source and slides piston member 220 with a screw mechanism (not shown).

Load cell 119 is also contained in syringe-driving mechanism 117. It detects the pressure applied to piston member 220 by syringe-driving mechanism 117. Injection head 110 also has sub touch panel 121 placed in the forward section of the upper surface. Various guidance messages and the like are displayed on sub touch panel 121.

In chemical solution injector 100 of the embodiment, injection head 110 includes inclination detection sensor 122 formed of an acceleration sensor or the like, serving as an attitude-determining means . Inclination detection sensor 122 determines the attitude of injection head 110. Injection head 110 is supported to be pivotable vertically in the injection attitude in which the leading end is located below the trailing end as shown in Fig. 6(a), and, in the preparation attitude in which the leading end is located below the trailing end as shown in Fig. 6(b). Inclination detection sensor 122 detects the direction of gravity acting on itself, for example, to detect whether injection head 110 is in the injection attitude or the preparation attitude.

Injection head 110 as described above is wire-connected to injection control unit 101 which controls the operation of injection head 110. Thus, as shown in Fig. 2, injection control unit 101 contains computer unit 130 and is wire-connected to imaging control unit 402 of CT angiography apparatus 400 through communication network 404. Injection control unit 101 has operation panel 103, main touch panel 104, speaker unit 105 and the like, all of which are disposed on the front face of unit housing 106. The abovementioned various devices are connected to computer unit 130.

Computer unit 130 is formed of a so-called one-chip microcomputer provided with hardware such as CPU (Central Processing Unit) 131, ROM (Read Only Memory) 132, RAM (Random Access Memory) 133, I/F (Interface) 134 and the like. Computer unit 130 has an appropriate computer program installed as firmware or the like on an information storage medium such as ROM 132, and CPU 131 executes various types of processing in accordance with the computer program.

In chemical solution injector 100 of the embodiment, computer unit 130 operates in accordance with the computer program installed as described above to logically have various means such as chemical solution sucking means 141, bubble-removing means 142, chemical solution injecting means 143, initial setting means 144, removal-control means 145, injection-control means 146, and drive-regulating means 147, as shown in Fig. 1.

Chemical solution sucking means 141 corresponds to the function of CPU 131 which controls the operation of syringe-driving mechanism 117 in accordance with the computer program in ROM 132 and entry-operation on sub touch panel 121. To suck a chemical solution from a chemical solution tank (not shown) into chemical solution syringe 200, chemical solution sucking means 141 causes piston member 220 of chemical solution syringe 200 mounted on injection head 110 to be pulled out of cylinder member 210 for a predetermined distance.

Though there are the pre-filled type and the refill type in chemical solution syringe 200 as described above, basically, only the refill type of chemical solution syringe 200 is used in chemical solution injector 100 of the embodiment. New empty chemical solution syringe 200 is connected to the chemical solution tank filled with a chemical solution through a suction tube (not shown). This chemical solution syringe 200 is mounted on chemical solution injector 100, and piston member 220 thereof is pulled from the leading end to near the trailing end of cylinder member 210 by syringe-driving mechanism 117 at a predetermined speed and predetermined pressure, in accordance with entry-operation on sub touch panel 121, thereby the chemical solution will be sucked into chemical solution syringe 200 from the solution tank.

Bubble-removing means 142 corresponds to the function of CPU 131 which controls the operation of syringe-driving mechanism 117 in accordance with the computer program and entry-operation. It presses piston member 220 into cylinder member 210 for a predetermined distance in order to remove bubbles from the chemical solution in chemical solution syringe 200.

Chemical solution injecting means 143 corresponds to the function of CPU 131 which controls the operation of syringe-driving mechanism 117 in accordance with the computer program and entry-operation. It presses piston member 220 into cylinder member 210 in order to inject the chemical solution into a patient from chemical solution syringe 200.

More specifically, for bubble-removing, piston member 220 is pressed into cylinder member 210 at a predetermined speed and predetermined pressure only for a predetermined distance of several millimeters necessary for removing bubbles from the chemical solution in chemical solution syringe 200 in accordance with entry-operation on sub touch panel 121. On the other hand, for chemical solution injection, piston member 220 is pressed into cylinder member 210 based on the injection speed, injection pressure, injection capacity and the like previously entered to main touch panel 104, for example.

In chemical solution injector 100 of the embodiment, for example, a suction switch and a removal switch are displayed on sub touch panel 121, and an injection switch is displayed on main touch panel 104 (the switches are not shown). Entry-operation on the suction switch causes the abovementioned chemical solution suction to be performed. Entry-operation on the removal switch causes the abovementioned bubble-removing to be performed. Entry-operation on the injection switch causes the abovementioned chemical solution injection to be performed.

Initial setting means 144 corresponds to the function of CPU 131 which controls the operation of syringe-driving mechanism 117 in accordance with the computer program and data detected by mount/demount detection sensor 120. When the mounting of chemical solution syringe 200 is detected by mount/demount detection sensor 120, initial setting means 144 sets the bubble-removing means 142 and chemical solution injecting means 143 inoperable, whereas only the chemical solution sucking means 141 operable.

The following corresponds to the function of CPU 131 which controls the operation of syringe-driving mechanism 117 in accordance with the computer program and data detected by inclination detection sensor 122. Removal-control means 145 causes chemical solution sucking means 141 and chemical solution injecting means 143 to be inoperable and causes bubble-removing means 142 to be operable, when the operation of chemical solution sucking means 141 is completed and then it is determined that injection head 110 is positioned in the preparation attitude.

Injection-control means 146 causes chemical solution sucking means 141 and bubble-removing means 142 to be inoperable and causes chemical solution injecting means 143 to be operable when the operations of chemical solution sucking means 141 and bubble-removing means 142 are completed in order in the preparation attitude and then it is determined that injection head 110 is positioned in the injection attitude. Drive-regulating means 147 always monitors data detected by mount/demount detection sensor 120, and if the mounting of chemical solution syringe 200 is not detected, it deactivates syringe-driving mechanism 117 regardless of the operations of means 141 to 143.

Although the abovementioned various means of chemical solution injector 100 are accomplished by pieces of hardware such as main/sub touch panels 104, 121 as required, they are mainly implemented by CPU 131 as a piece of hardware functioning in accordance with the resources and the computer program stored on an information storage medium such as ROM 132.

The computer program is stored on an information storage medium such as ROM 132 as software for causing CPU 131 to control the operation of syringe-driving mechanism 117 in accordance with entry-operation on main/sub touch panels 104, 121, data detected by mount/demount detection sensor 120 and inclination detection sensor 122 and the like, for example.

### [Operation of the Embodiment]

When chemical solution injector 100 of the embodiment is used in the abovementioned structure, chemical solution injector 100 is disposed near imaging diagnostic unit 401 of CT angiography apparatus 400 as shown in Fig. 5, and new empty chemical solution syringe 200, pressure-resistant cover 300, the chemical solution tank filled with a chemical solution, a suction tube, an injection tube (not shown) and the like are prepared for use.

Next, as shown in Figs. 3 and 4, chemical solution syringe 200 is mounted on injection head 110 of chemical solution injector 100 together with pressure-resistant cover 300. Chemical solution syringe 200 is connected to the chemical solution tank through the suction tube. It should be noted that the mounting and demounting of chemical solution syringe 200 on and from injection head 110 and the connection and disconnection of the suction tube to and from chemical solution syringe 200 as described above are usually performed while injection head 110 is positioned in the injection attitude as shown in Fig. 6(a) for convenience of the operation.

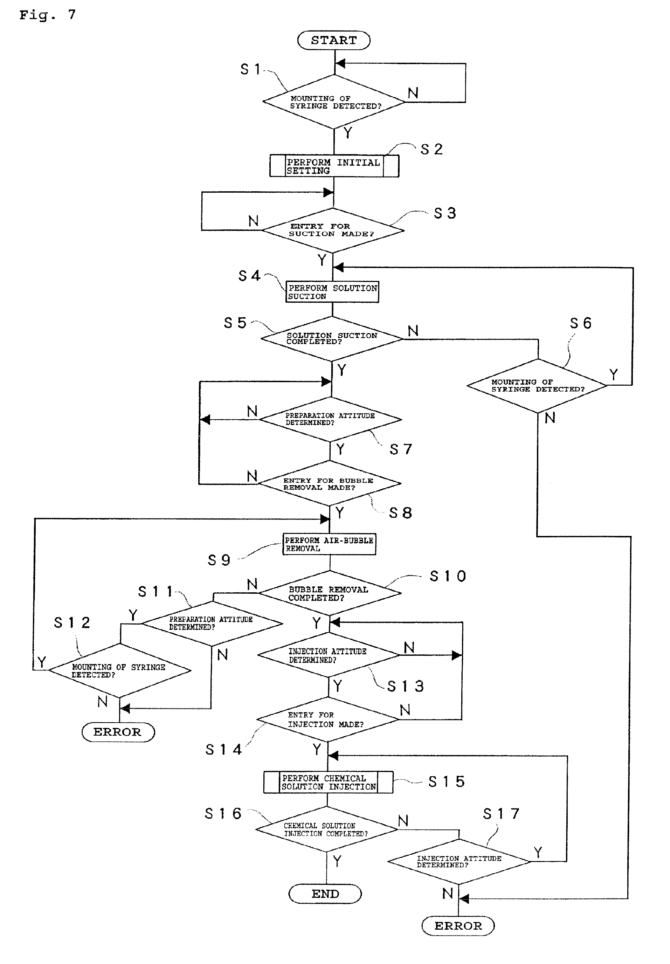

In chemical solution injector 100 of the embodiment, when chemical solution syringe 200 is mounted on injection head 110 together with pressure-resistant cover 300, mount/demount detection sensor 120 detects the mounting of chemical solution syringe 200 (step S1) as shown in Fig. 7, and then the initial setting is made in response to the detection (step S2).

In chemical solution injector 100 of the embodiment, the suction, removal, and injection switches for performing the chemical solution suction, bubble-removing, and chemical solution injection, respectively, are displayed on main/sub touch panels 104, 121 as described above. However, the bubble-removing or chemical solution injection is not performed, in the abovementioned initial setting state.

In chemical solution injector 100 of the embodiment, when chemical solution syringe 200 is mounted and the initial setting is made as described above, an operator is notified of the detection of the mounting of chemical solution syringe 200, the completion of the initial setting, the fact that the chemical solution suction, bubble-removing, and chemical solution injection are not performed yet, and the need to perform the chemical solution suction first, for example, through message display or the like on main/sub touch panels 104, 121.

Then, for example, the operator connects chemical solution syringe 200 on injection head 110 in the injection attitude to the chemical solution tank through the suction tube. Then, the operator makes entry on the suction switch (step S3), thereby the chemical solution suction is performed (steps S4 to S6). In the chemical solution suction, piston member 220 is pulled from the leading end to near the trailing end of cylinder member 210 by cylinder driving mechanism 117 at a predetermined speed and predetermined pressure, and then the chemical solution is sucked from the chemical solution tank into chemical solution syringe 200.

During the abovementioned chemical solution suction, the mounting of chemical solution syringe 200 is always monitored (step S6). If the detection is stopped, the occurrence of an error is determined. In this case, for example, driving mechanism 117 is forcedly stopped. The operator is notified of the occurrence of the error through message display or the like on main/sub touch panels 104, 121.

On the other hand, when the abovementioned chemical solution suction is completed (step S5) and then injection head 110 is positioned in the preparation attitude (step S7), only bubble-removing can be performed (steps S8, S9). In this case, for example, the operator is notified of the completion of the chemical solution suction, and ,the fact that bubble-removing should be performed with injection head 110 positioned in the preparation attitude, on main/sub touch panels 104, 121.

Then, the operator disconnects the suction tube from chemical solution syringe 200 on injection head 110 in the injection attitude, alternatively connects the injection tube thereto. And the operator sets injection head 110 in the preparation attitude, and then makes entry on the removal switch (step S8). In response to the entry-operation, the bubble-removing is performed (steps S9 to S12).

In the bubble-removing, piston member 220 is pressed into cylinder member 210 only several millimeters at a predetermined speed and predetermined pressure by syringe-driving mechanism 117, thereby bubbles are removed from chemical solution syringe 200, and the injection tube is filled with the chemical solution. Since the chemical solution syringe 200 is positioned in the preparation attitude together with injection head 110, bubbles are favorably removed from chemical solution syringe 200 and the injection tube.

During the bubble-removing, the preparation attitude of injection head 110 and the mounting of chemical solution syringe 200 are always monitored (steps S11, S12). If at least one of them will not be detected, the occurrence of an error is determined. In this case, for example, driving mechanism 117 is forcedly stopped, and the occurrence of the error is notified through message display or the like on main/sub touch panels 104, 121. When injection head 110 is turned from the preparation attitude during the bubble-removing, or chemical solution syringe 200 comes off during the bubble-removing, the bubble-removing is stopped and the operator is notified of the occurrence of the error.

On the other hand, when the abovementioned bubble-removing is completed (step S10), the operator is notified of the completion of the bubble-removing and the need to set injection head 110 in the injection attitude to perform chemical solution injection, for example, on main/sub touch panels 104, 121. Then, the operator connects the injection tube filled with the chemical solution to a blood vessel of a patient and turns injection head 110 from the preparation attitude to the injection attitude as shown in Figs. 6(a) and 6(b).

The injection attitude of injection head 110 is detected by inclination detection sensor 122 (step S13). In response to the detection, only the injection can be performed (steps S14, S15). For example, the operator is notified of the fact that the chemical solution injection can be performed on main/sub touch panels 104, 121.

When the operator makes entry on the injection switch (step S14), piston member 220 is pressed into cylinder member 210 in accordance with a predetermined injection speed, the injection pressure, the injection capacity and the like, previously entered on main touch panel 104 by the operator, for example. In this manner, the chemical solution is injected into the patient from chemical solution syringe 200 (step S15).

Since chemical solution syringe 200 is positioned in the injection attitude together with injection head 110, even if small bubbles are present in the chemical solution syringe 200, they are not injected into the patient. During the chemical solution injection, the injection attitude of injection head 110 and the mounting of chemical solution syringe 200 are always monitored (steps S16, S17). If the detection of at least one of them is stopped, the occurrence of an error is determined.

In this case, for example, driving mechanism 117 is forcedly stopped and the occurrence of the error is notified through message display or the like on main/sub touch panels 104, 121. If injection head 110 is turned from the injection attitude, or, chemical solution syringe 200
comes off during the chemical solution injection, the chemical solution injection is stopped, and the operator is notified of the occurrence of the error.

In imaging diagnostic system 1000 of the embodiment, chemical solution injector 100 and CT angiography apparatus 400 communicate with each other to associate their operations. For this reason, the imaging in CT angiography apparatus 400 is not started until the start of chemical solution injection in chemical solution injector 100 as described above. For example, CT angiography apparatus 400 automatically starts operation after the lapse of a predetermined time period after the start of chemical solution injection in chemical solution injector 100.

### [Effect of the Embodiment]

In imaging diagnostic system 1000 of the embodiment, as described above, only the chemical solution suction can be performed for chemical solution syringe 200 in the initial state, and after the chemical solution suction is completed and injection head 110 is positioned in the preparation attitude, only the bubble-removing can be performed. After the bubble-removing is completed and injection head 110 is positioned in the injection attitude, only the chemical solution injection can be performed.

This can automatically prevent the bubble-removing from being performed in chemical solution syringe 200 which has not sucked a sufficient amount of chemical solution, and prevent the chemical solution injection from being performed in chemical solution syringe 200 which contains bubbles not completely removed, without requiring an additionally provided release switch or an increased burden in operation.

The position of injection head 110 in the preparation attitude is monitored during the bubble-removing, and the position of injection head 110 in the injection attitude is monitored during the chemical solution injection. If a change in the attitude is detected, the abovementioned operation is forcedly stopped. This can automatically prevent the bubble-removing and the chemical solution injection from being performed in an inappropriate attitude.

The mounting and demounting of chemical solution syringe 200 are detected. When the mounting of chemical solution syringe 200 is detected, the initial setting is made such that the bubble-removing or chemical solution injection cannot be performed, so that the proper initial setting is performed. The mounting of chemical solution syringe 200 is monitored during the chemical solution injection, the bubble removing, and the chemical solution injection, and if the detection is stopped, the operation performed at that time is forcedly stopped. This can automatically prevent the operation from being continued after chemical solution syringe 200 comes off, for example.

Since sub touch panel 121 for receiving entry to perform the chemical solution suction or the bubble-removing in chemical solution injector 100 is provided for injection head 110 on which chemical solution syringe 200 is mounted, the operator who makes entry to perform the chemical solution suction and the bubble-removing can reliably check the status of chemical solution suction and the bubble-removing.

In imaging diagnostic system 1000 of the embodiment, the chemical solution injection in chemical solution injector 100 is automatically associated with the imaging in CT angiography apparatus 400, so that the diagnostic images can be taken at the appropriate time from the patient injected with the contrast medium.

### [Modifications of the Embodiment]

The present invention is not in any way limited to the abovementioned embodiment, but various changes and modifications may be made therein without departing from the scope of the invention. In the above embodiment, illustrative chemical solution injector 100 performs automatic control such that the chemical solution suction, the bubble-removing, and the chemical solution injection are performed in order in chemical solution syringe 200 of the refill type. Alternatively, for example, it is possible to provide a chemical solution injector (not shown) which performs automatic control such that only bubble-removing and chemical solution injection are performed in order, in a chemical solution syringe of a refill type or a pre-filled type.

In the chemical solution injector which automatically controls only the bubble-removing and the chemical solution injection in the chemical solution syringe of the refill type, for example, it is preferable to perform chemical solution suction in the chemical solution syringe by using a dedicated chemical solution suction apparatus (not shown), or, to manually perform chemical solution suction as a step which is not automatically controlled.

In the above embodiment, chemical solution injector 100 uses only chemical solution syringe 200 of the refill type. However, it is possible to implement a chemical solution injector in which both of a chemical solution syringe of the refill type and a chemical solution syringe of the pre-filled type are used, for example (not shown). When such a chemical solution injector is implemented, an RFID (Radio Frequency Identification) chip (not shown) having data recorded thereon for representing the refill type or pre-filled type is put on the chemical solution syringe, and an RFID reader (not shown) for reading the data on the RFID chip is mounted as a syringe-determining means on the chemical solution injector.

It is determined whether the chemical solution syringe mounted on the injection head is of the pre-filled type or refilled type. In the chemical solution syringe of the pre-filled type, when the injection head is positioned in the preparation attitude after the initial state, only bubble-removing can be performed. When the injection head is located in the injection attitude after the completion of the bubble-removing, only chemical solution injection can be performed.

In the chemical solution syringe of the refill type, only chemical solution suction can be performed in the initial state. When the injection head is positioned in the preparation attitude after the completion of the chemical solution suction, only bubble-removing can be performed. When the injection head is located in the injection attitude after the completion of the bubble-removing, only chemical solution injection can be performed.

In such a chemical solution injector, it is possible to automatically prevent the chemical solution injection in the chemical solution syringe of the pre-filled type for which the bubble-removing is not completed, the bubble-removing in the chemical solution syringe of the refill type for which the chemical solution suction is not completed, and the chemical solution injection in the chemical solution syringe of the refill type for which the bubble-removing is not completed, without requiring an additionally provided release switch or an increased burden in operation.

Systems as described above in which the RFID chip is put on the chemical solution syringe and the RFID reader is mounted on the chemical solution injector have been invented and applied by the present applicant as Japanese Patent Application No. 2004-059034, Japanese Patent Application No. 2004-207345, Japanese Patent Application No. 2004-327479, Japanese Patent Application No. 2004-334942, Japanese Patent Application No. 2004-345493 and the like.

To simplify the description, the chemical solution suction is immediately performed for chemical solution syringe 200 of the refill type in the initial state in the above embodiment. In currently used typical chemical solution syringe 200 of the refill type, however, piston member 220 is located near the trailing end of cylinder member 210 in the initial state. It is necessary to make preparation for suction by moving piston member 220 to near the leading end of cylinder member 210 prior to chemical solution suction.

In chemical solution injector 100 in which such chemical solution syringe 200 is used, it is preferable that only the preparation for suction can be performed in the initial state, chemical solution suction can be performed after the completion of the preparation for suction, and bubble-removing can be performed in the preparatory attitude after the completion of the chemical solution suction.

In the above embodiment, to facilitate the description, the bubble-removing with chemical solution injector 100 is performed for chemical solution syringe 200 and the injection tube at the same time. Alternatively, it is possible that the bubble-removing for chemical solution injector 100 is performed only for chemical solution syringe 200, and the bubble-removing for the injection tube is performed by a dedicated exhaust valve.

In the above embodiment, inclination detection sensor 122 serving as the attitude-determining means for determining whether injection head 110 is positioned in the preparation attitude or the injection attitude is formed of an acceleration sensor or the like. Such an attitude-determining means may be realized by various structures, for example, a device for detecting the orientation by itself such as a mercury switch, an orientation sensor, and a magnetic sensor, and a device for detecting a relative angle to movable arm 112 such as a rotary encoder (not shown).

In the above embodiment, after entry-operation is made on the dedicated switch, a series of the chemical solution suction and bubble-removing is performed automatically in accordance with the registered data or the like. For example, the chemical solution suction and bubble-removing may be manually operated in real time. In such a chemical solution injector (not shown), a manual-operating means is formed of a manual switch for allowing entry-operation to move syringe-driving mechanism 117 forward and rearward, and chemical solution injecting means 141 for automatically performing the chemical solution injection is initially set to be inoperable by initial setting means 144, by way of example.

For performing the chemical solution suction and the bubble-removing, only the chemical solution suction can be performed in the initial state in real time based on entry-operation to the manual-operating means, and when injection head 110 is positioned in the preparation attitude after the completion of the chemical solution suction, the bubble-removing can be performed in real time in accordance with entry-operation to the manual-operating means. For performing the bubble-removing without performing the chemical solution suction, the bubble-removing can be performed in real time in accordance with entry-operation to the manual-operating means when injection head 110 is positioned in the preparation attitude after the initial state.

In this case, however, a series of the chemical solution suction and bubble-removing is not performed, so that the completion thereof cannot be automatically detected simply. Thus, the completions of the chemical solution suction and the bubble-removing are individually detected on the basis of the moving distance or the position of syringe-driving mechanism 117, and chemical solution injecting means 144 can be activated when it is determined that injection head is positioned in the injection attitude after the completions of the chemical solution suction and bubble-removing are detected. In such a chemical solution injector, the chemical solution suction and bubble-removing can be manually operated as desired by an operator, and the completions of the chemical solution suction and bubble-removing can be automatically detected to cancel the deactivation of chemical solution injecting means 144.

When the chemical solution syringe of the pre-filled type or refill type is mounted on such a chemical solution injector, it is preferable that it is determined whether the chemical solution syringe mounted on injection head 110 is of the pre-filled type or refill type, and when it is determined that the chemical solution syringe of the pre-filled type is mounted, the chemical solution injecting means can be activated after the detection of the completion of the bubble-removing, and when it is determined that the chemical solution syringe of the refill type is mounted, the chemical solution injecting means can be activated after the detection of the completions of the chemical solution suction and bubble-removing in order.

In such a chemical solution injector, it is preferable that syringe-driving mechanism 117 performs only the bubble-removing in accordance with entry-operation when it is determined that the chemical solution syringe of the pre-filled type is mounted, and syringe-driving mechanism 117 performs the chemical solution suction and bubble-removing in order in accordance with entry-operation when it is determined that the chemical solution syringe of the refill type is mounted.

In the above embodiment, chemical solution injector 100 includes one syringe-driving mechanism 117 and injects only the contrast medium. It is also possible to implement a chemical solution injector (not shown) which includes two syringe-driving mechanisms 117 to inject a contrast medium and physiological saline or a chemical solution injector (not shown) which includes three or more syringe-driving mechanisms 117 to inject three or more types of chemical solutions.

In the above embodiment, CT angiography apparatus 400 is used as the imaging diagnostic apparatus, and chemical solution injector 100 injects the contrast medium for CT. For example, it is possible that an MRI apparatus or a PET apparatus is used as the imaging diagnostic apparatus and the chemical solution injector injects a contrast medium therefor.

In the above embodiment, CPU 131 operates in accordance with the computer program stored in RAM 133 or the like to realize logically various means as various functions of chemical solution injector 100. Each of the various means may be formed as specific hardware, or some of them may be stored as software in RAM 133 or the like, while others may be formed as hardware.

### Industrial Availability

The present invention is used, for example, in a chemical solution injection system for injecting a contrast medium into a patient with an imaging diagnostic apparatus such as a CT (Computed Tomography) scanner.

## Claims

1. A chemical solution injector for injecting a chemical solution into a patient from a chemical solution syringe by relatively moving a cylinder member and a piston member of the chemical solution syringe, the cylinder member having a conduit portion formed at its leading end and an opening formed at its trailing end, the piston member being slidably inserted into the cylinder member through the opening, the chemical solution injector comprising:
an injection head on which the chemical solution syringe is removably mounted;
a syringe-driving mechanism for relatively moving the cylinder member and the piston member of the chemical solution syringe mounted on the injection head;
a head support mechanism for supporting the injection head movable to an injection attitude in which a leading end of the chemical solution syringe is located below a trailing end thereof, and to a preparation attitude in which the leading end is located above the trailing end;
an attitude-determining means for determining whether the injection head is positioned in the injection attitude or the preparation attitude;
a bubble-removing means for pressing the piston member into the cylinder member for a predetermined distance under control of the syringe-driving mechanism to remove bubbles from the chemical solution in the chemical solution syringe;
a chemical solution injecting means for pressing the piston member into the cylinder member under control of the syringe-driving mechanism to inject the chemical solution into the patient from the chemical solution syringe ;
an initial setting means for performing initial setting such that the bubble-removing means and the chemical solution injecting means are inoperable;
a removal-control means for causing the bubble-removing means to be operable while the chemical solution injecting means is held inoperable, when it is determined that the injection head is positioned in the preparation attitude after the completion of the initial setting; and
an injection-control means for causing the bubble-removing means to be inoperable and causing the chemical solution injecting means to be operable, when it is determined that the injection head is positioned in the injection attitude after the completion of the operation of the bubble-removing means in the preparation attitude.

2. A chemical solution injector for injecting a chemical solution into a patient from a chemical solution syringe by relatively moving a cylinder member and a piston member of the chemical solution syringe after suction of the chemical solution into the chemical solution syringe from a chemical solution tank, the cylinder member having a conduit portion formed at its leading end and an opening formed at its trailing end, the piston member being slidably inserted into the cylinder member through the opening, the chemical solution injector comprising :
an injection head on which the chemical solution syringe is removably mounted;
a syringe-driving mechanism for relatively moving the cylinder member and the piston member of the chemical solution syringe mounted on the injection head;
a head support mechanism for supporting the injection head movable to an injection attitude in which a leading end of the chemical solution syringe is located below a trailing end thereof, and to a preparation attitude in which the leading end is located above the trailing end;
an attitude-determining means for determining whether the injection head is positioned in the injection attitude or the preparation attitude;
a chemical solution sucking means for pulling the piston member from the cylinder member for a predetermined distance under control of the syringe-driving mechanism to suck the chemical solution into the chemical solution syringe from the chemical solution tank;
a bubble-removing means for pressing the piston member into the cylinder member for a predetermined distance under control of the syringe-driving mechanism to remove bubbles from the chemical solution in the chemical solution syringe;
a chemical solution injecting means for pressing the piston member into the cylinder member under control of the syringe-driving mechanism to inject the chemical solution into the patient from the chemical solution syringe;
an initial setting means for performing initial setting such that the bubble-removing means and the chemical solution injecting means are inoperable;
a removal-control means for causing the chemical solution sucking means and the chemical solution injecting means to be inoperable and the bubble-removing means to be operable, when it is determined that the injection head is positioned in the preparation attitude after the completion of the operation of the chemical solution sucking means; and
an injection-control means for causing the chemical solution sucking means and the bubble-removing means to be inoperable and the chemical solution injecting means to be operable, when it is determined that the injection head is positioned in the injection attitude after the operation of the chemical solution sucking means and the operation of the bubble-removing means are completed in order.

3. A chemical solution injector for at least injecting a chemical solution into a patient by relatively moving a cylinder member and a piston member of the chemical solution syringe after suction of the chemical solution into the chemical solution syringe from a chemical solution tank, the cylinder member having a conduit portion formed at its leading end and an opening formed at its trailing end, the piston member being slidably inserted into the cylinder member through the opening, the chemical solution injector comprising:
an injection head on which a pre-filled type syringe filled with the chemical solution or a refill type syringe into which the chemical solution is sucked from an external chemical solution tank are selectively and removably mounted;
a syringe-determining means for determining whether the chemical solution syringe mounted on the injection head is of the pre-filled type or the refill type;
a syringe-driving mechanism for relatively moving the cylinder member and the piston member of the chemical solution syringe mounted on the injection head;
a head support mechanism for supporting the injection head movable to an injection attitude in which a leading end of the chemical solution syringe is located below a trailing end thereof and to a preparation attitude in which the leading end is located above the trailing end;
an attitude-determining means for determining whether the injection head is positioned in the injection attitude or the preparation attitude;
a chemical solution sucking means for pulling the piston member from the cylinder member for a predetermined distance under control of the syringe-driving mechanism to suck the chemical solution into the chemical solution syringe of the refill type from the chemical solution tank;
a bubble-removing means for pressing the piston member into the cylinder member for a predetermined distance under control of the syringe-driving mechanism to remove bubbles from the chemical solution in the chemical solution syringe ;
a chemical solution injecting means for pressing the piston member into the cylinder member under control of the syringe-driving mechanism to inject the chemical solution into the patient from the chemical solution syringe;
an initial setting means for performing initial setting such that the bubble-removing means and the chemical solution injecting means are inoperable;
a pre-filled removing means for causing the bubble-removing means to be operable while the chemical solution injecting means and the chemical solution sucking means are held inoperable when it is determined that the injection head is positioned in the preparation attitude and that the chemical solution syringe is of the pre-filled type after the completion of the initial setting;
a pre-filled injecting means for causing the chemical solution sucking means and the bubble-removing means to be inoperable and the chemical solution injecting means to be operable, when it is determined that the injection head is positioned in the injection attitude and that the chemical solution syringe is of the pre-filled type after the completion of the operation of the bubble-removing means in the preparation means;
a refill sucking means for first causing the chemical solution injecting means and the bubble-removing means to be inoperable and the chemical solution sucking means to be operable, when it is determined that the chemical solution syringe is of the refill type after the completion of the initial setting;
a refill removing means for causing the bubble-removing means to be operable, the chemical solution injecting means is held inoperable, when it is determined that the injection head is positioned in the preparation attitude and that the chemical solution syringe is of the refill type after the completion of the operation of the chemical solution sucking means; and
a refill injecting means for causing the chemical solution sucking means and the bubble-removing means to be inoperable and the chemical solution injecting means to be operable when it is determined that the injection head is positioned in the injection attitude and that the chemical solution syringe is of the refill type after the operation of the chemical solution sucking means and the operation of the bubble-removing means are completed.

4. A chemical solution injector for injecting a chemical solution into a patient from a chemical solution syringe by relatively moving a cylinder member and a piston member of the chemical solution syringe, the cylinder member having a conduit portion formed at its leading end and an opening formed at its trailing end, the piston member being slidably inserted into the cylinder member through the opening, the chemical solution injector comprising:
an injection head on which the chemical solution syringe is removably mounted;
a syringe-driving mechanism for relatively moving the cylinder member and the piston member of the chemical solution syringe mounted on the injection head;
a head support mechanism for supporting the injection head movable to an injection attitude in which a leading end of the chemical solution syringe is located below a trailing end thereof and to a preparation attitude in which the leading end is located above the trailing end;
an attitude-determining means for determining whether the injection head is positioned in the injection attitude or the preparation attitude;
a chemical solution injecting means for pressing the piston member into the cylinder member under control of the syringe-driving mechanism to inject the chemical solution into the patient from the chemical solution syringe;
an initial setting means for performing initial setting such that the chemical solution injecting means is inoperable;
a manual-operating means for receiving entry-operation to cause the syringe-driving mechanism to perform at least bubble-removing of removing bubbles from the chemical solution in the chemical solution syringe in real time;
an operation-driving means for operating the syringe-driving mechanism in real time in response to the entry-operation to the manual-operating means when it is determined that the injection head is positioned in the preparation attitude;
a completion detecting means for detecting the completion of the bubble-removing based on at least one of a moving distance and a position of the syringe-driving mechanism when it is determined that the injection head is positioned in the preparation attitude ; and
an injection-control means for causing the chemical solution injecting means to be operable when it is determined that the injection head is positioned in the injection attitude after the completion of the bubble-removing is detected.

5. A chemical solution injector for injecting a chemical solution into a patient from a chemical solution syringe by relatively moving a cylinder member and a piston member of the chemical solution syringe after suction of the chemical solution into the chemical solution syringe from a chemical solution tank, the cylinder member having a conduit portion formed at its leading end and an opening formed at its trailing end, the piston member being slidably inserted into the cylinder member through the opening, the chemical solution injector comprising:
an injection head on which the chemical solution syringe is removably mounted;
a syringe-driving mechanism for relatively moving the cylinder member and the piston member of the chemical solution syringe mounted on the injection head;
a head support mechanism for supporting the injection head movable to an injection attitude in which a leading end of the chemical solution syringe is located below a trailing end thereof and to a preparation attitude in which the leading end is located above the trailing end;
an attitude-determining means for determining whether the injection head is positioned in the injection attitude or the preparation attitude;
a chemical solution injecting means for pressing the piston member into the cylinder member under control of the syringe-driving mechanism to inject the chemical solution into the patient from the chemical solution syringe;
an initial setting means for performing initial setting such that the chemical solution injecting means is inoperable;
a manual-operating means for receiving entry-operation to cause the syringe-driving mechanism to perform at least chemical solution suction of sucking the chemical solution into the chemical solution syringe from the chemical solution tank and bubble-removing of removing bubbles from the chemical solution in the chemical solution syringe in real time;
an operation-driving means for operating the syringe-driving mechanism in real time in response to the entry-operation to the manual-operating means;
a suction-detecting means for detecting the completion of the chemical solution suction based on at least one of a moving distance and a position of the syringe-driving mechanism after the completion of the initial setting;
a removal detecting means for detecting the completion of the bubble-removing based on at least one of a moving distance and a position of the syringe-driving mechanism when it is determined that the injection head is positioned in the preparation attitude after the completion of the chemical solution suction; and
a injection-control means for causing the chemical solution injecting means to be operable when it is determined that the injection head is positioned in the injection attitude after the completion of the chemical solution suction and the completion of the bubble-removing are detected in order.

6. A chemical solution injector for at least injecting a chemical solution into a patient by relatively moving a cylinder member and a piston member of the chemical solution syringe after suction of the chemical solution into the chemical solution syringe from a chemical solution tank, the cylinder member having a conduit portion formed at its leading end and an opening formed at its trailing end, the piston member being slidably inserted into the cylinder member through the opening, the chemical solution injector comprising:
an injection head on which the chemical solution syringe of a pre-filled type previously filled with the chemical solution or the chemical solution syringe of a refill type into which the chemical solution is sucked from an external chemical solution tank are selectively and removably mounted;
a syringe-driving mechanism for relatively moving the cylinder member and the piston member of the chemical solution syringe mounted on the injection head;
a head support mechanism for supporting the injection head movable to an injection attitude in which a leading end of the chemical solution syringe is located below a trailing end thereof and to a preparation attitude in which the leading end is located above the trailing end;
an attitude-determining means for determining whether the injection head is positioned in the injection attitude or the preparation attitude;
a chemical solution injecting means for pressing the piston member into the cylinder member under control of the syringe-driving mechanism to inject the chemical solution into the patient from the chemical solution syringe;
an initial setting means for performing initial setting such that the chemical solution injecting means is inoperable;
a manual-operating means for receiving entry-operation to cause the syringe-driving mechanism to perform at least chemical solution suction of sucking the chemical solution into the chemical solution syringe from the chemical solution tank and bubble-removing of removing bubbles from the chemical solution in the chemical solution syringe in real time;
a operation-driving means for operating the syringe-driving mechanism in real time in response to the entry-operation to the manual-operating means;
a suction-detecting means for detecting the completion of the chemical solution suction based on at least one of a moving distance and a position of the syringe-driving mechanism, after the completion of the initial setting;
a removal detecting means for detecting the completion of the bubble-removing based on at least one of a moving distance and a position of the syringe-driving mechanism when it is determined that the injection head is positioned in the preparation attitude after the completion of the chemical solution suction;
a syringe-determining means for determining whether the chemical solution syringe mounted on the injection head is of the pre-filled type or the refill type; and
an injection-control means for causing the chemical solution injecting means to be operable after the completion of the bubble-removing is detected when it is determined that the chemical solution syringe is of the pre-filled type, and causing the chemical solution injecting means to be operable after the completion of the chemical solution suction and the completion of the bubble-removing are detected in order, when it is determined that the chemical solution syringe is of the refill type.

7. The chemical solution injector according to claim 6, wherein the operation-driving means causes the syringe-driving mechanism to perform only the bubble-removing in response to the entry-operation when it is determined that the chemical solution syringe is pre-filled type, and
the operation-driving means causes the syringe-driving mechanism to perform the chemical solution suction and the bubble-removing in order in response to the entry-operation when it is determined that the chemical solution syringe is refill type.

8. The chemical solution injector according to any one of claims 5, 6, and 7, wherein in the chemical solution syringe for the chemical solution suction, the piston member is positioned near the trailing end of the cylinder member in the initial state,
the manual-operating member receives entry-operation of preparation for suction in which the piston member of the syringe for the chemical solution suction is moved from near the trailing end of the cylinder member to near the leading end thereof,
the chemical solution injector further comprising a preparation-detecting means for detecting the completion of the preparation for suction, based on at least one of a moving distance and a position of the syringe-driving mechanism after the initial setting,
wherein the suction-detecting means detects the completion of the chemical solution suction, based on at least one of a moving distance and a position of the syringe driving mechanism after the preparation for suction, and
the injection-control means does not cause the chemical solution injecting means to be operable, unless the completion of the preparation for suction and the completion of the chemical solution suction are detected in order.

9. The chemical solution injector according to any one of claims 4 to 8, wherein the manual-operating means is provided in the injection head.

10. The chemical solution injector according to claim 2 or 3,
wherein, in the chemical solution syringe for the chemical solution suction, the piston member is positioned near the trailing end of the cylinder member in the initial state,
the chemical solution injector further comprising a suction-preparing means which presses the piston member into the cylinder member for a predetermined distance by operation of the syringe-driving mechanism for sucking preparation in which the piston member of the chemical solution syringe for chemical solution suction will be positioned near the leading end of the cylinder member of the chemical solution syringe for chemical solution suction.

11. The chemical solution injector according to any one of claims 1 to 10, further comprising a mount/demount detecting means for detecting the mounting of chemical solution syringe on the injection head,
wherein the initial setting means performs the initial setting when the mounting of the chemical solution syringe is detected.

12. The chemical solution injector according to any one of claims 1 to 10, further comprising:
a mount/demount detecting means for detecting the mounting of chemical solution syringe; and
a drive-regulating means for causing the syringe-driving mechanism to be inoperable while the mounting of the chemical solution syringe is not detected,
wherein the initial setting means performs the initial setting when the mounting of the chemical solution syringe is detected.
